# EUROPEAN PATENT SPECIFICATION

(11) **EP 1 636 295 B1**
(45) Date of publication and mention of the grant of the patent: **13.04.2011**
(21) Application number: 04748676.6
(22) Date of filing: 23.06.2004
(51) Int. Cl.: C08J 3/28, A61K 9/00, A61K 47/30, B29C 71/04, B06B 3/00

(54) **DRUG DELIVERY DEVICE COMPRISING AN ACTIVE COMPOUND AND METHOD FOR RELEASING AN ACTIVE COMPOUND FROM A DRUG DELIVERY DEVICE**
ARZNEISTOFFZUFUHRVORRICHTUNG, DIE EINEN WIRKSTOFF ENTHÄLT, UND VERFAHREN ZUR FREISETZUNG EINES WIRKSTOFFS AUS EINER ARZNEISTOFFZUFUHRVORRICHTUNG
DISPOSITIF D'ADMINISTRATION DE MEDICAMENTS COMPRENANT UN COMPOSE ACTIF ET PROCEDE DE LIBERATION DE CELUI-CI A PARTIR D'UN DISPOSITIF D'ADMINISTRATION DE MEDICAMENTS

(30) Priority: 23.06.2003 NL 1023720
(43) Date of publication of application: 22.03.2006
(73) Proprietor: Dolphys Medical B.V., 5612 AZ Eindhoven (NL)
(72) Inventor: BRUINEWOUD, Henny, NL-5612 DC Eindhoven (NL); KEURENTJES, Johannes, Theodorus, Faustinus, NL-5708 EH Helmond (NL); KEMMERE, Maria, Francisca, NL-5708 EH Helmond (NL)
(74) Representative: van Westenbrugge, Andries
(86) International application number: PCT/NL2004/000446
(87) International publication number: WO 2004/113422

(56) References cited:
- EP-A- 0 317 180
- WO-A-00/69942
- WO-A-03/017972
- US-A- 4 657 543
- US-A- 6 132 752
- US-A1- 2002 028 216
- DATABASE WPI Section Ch, Week 199440 Derwent Publications Ltd., London, GB; Class A96, AN 1994-322082 XP002275590 & JP 06 247841 A (NIPPON KAYAKU KK) 6 September 1994 (1994-09-06)

## Description

### Technical field of the invention

The present invention relates to a drug delivery device comprising a solid polymeric material having a barrier effect against diffusion of active compounds, wherein the transport characteristics of said material can be modified by using an external source. In particular, the present invention relates to a method for delivering a drug to a mammal in need thereof by employing a drug delivery device, in particular for implantation in a mammalian body, wherein the delivery of the drug from the drug delivery device is actively controlled by means of supplying energy from an external energy source. The drug delivery device is suitable for implantation in a mammalian body, for incorporation into a prosthesis or an artificial organ.

### Background of the invention

The fact that materials such as polymers can have a barrier effect against diffusion of an active compound is generally known It is also generally known that these transport characteristic can be modified.

One problem in connection with known methods for modifying the abovementioned transport characteristics is that these are usually irreversible in some respect or other. A change in the physical state, including the degree of dissolution of a solid material, can result in the transport characteristics of a material being modified. Insofar as the material can again be brought into the original physical state, the active compound has diffused out. In the state of the art there is therefore a need for a drug delivery device and a method with which, when this is employed, the abovementioned transport characteristics can be modified in a reversible manner, so that the diffusion of an active compound can not only be interrupted in a controlled manner but can also, if desired, be repeated reproducibly.

Several systems are known from the prior art. US 6.649.702 discloses a drug delivery system called "P-gel", said drug delivery system comprising stabilised P-triblock micelles. These micelles are made ofP-triblock polymers, e.g. PEO-PPO-PEO triblock polymers (PEO is polyethylene oxide; PPO is polypropylene oxide), wherein the terminal blocks (i.e. PEO) have hydrophilic character and the central block (i.e. PPO) has hydrophobic character. Such P-triblock copolymers are for example disclosed in US 5.516.703.

According to US 6.649.702, stabilisation of the P-triblock micelles is achieved by direct radical cross-linking, incorporation of oil or polymerisation of a temperature-responsive LCST ("Low Critical Solution Temperature") hydrogel, the latter system being indicated in US 6.649.702 as "P-gel" which may be obtained by polymerisation of N,N-diethylacrylamide in the presence of the P-triblock micelles. The stabilised P-triblock micelles can be loaded with hydrophobic drugs such as doxorubicin, wherein the hydrophobic drug can be released in a controlled and reversible manner by raising the temperature by using e.g. ultrasound, either constant wave or pulsed. Obviously, when administered to patients by e.g. IV injection such micelles circulate in the blood stream and are eventually metabolised thereby making them unsuitable as drug delivery devices where prolonged administration of the drug is required.

WO 03/017972 and JP A 6247841 disclose a drug delivery system similar to that of US 6.649.702.

The working mechanism of the drug delivery devices disclosed in US 6.649.702, WO 03/017972 and JP A 6247841 is based on a physical change of the device that is entropy driven since due to the higher temperature water molecules bound to the central hydrophobic groups are released (cf. for example WO 03/017972, page 6, lines 6 - 8).

US 6.312.708 discloses a system for controlled drug release of the neurotoxin botulinum toxin for a prolonged period of time. The system comprises a polymeric carrier comprising one or more biodegradable polymers and a stabilised botulinum toxin associated with the carrier. The system may be implanted subdermally, e.g. subcutaneously, intramuscularly and the like, or may be administered by injection. Preferably, the polymeric carrier is in the form of microspheres in which the botulinum toxin is incorporated, and the microspheres may be pressed into the form of a disc and implanted as a pellet. It is preferred that the polymeric carrier is comprised of nontoxic, non-immunological, biocompatible polymers such as poly(lactic acid), poly(glycolic acid) and the like. Such polymers are for example Medisorb® polymers which are said to be available from Medisorb Technologies International, now Alkermes, Inc. (cf. www.alkermes.com). Example 4 of US 6.312.708 discloses four sets of microspheres each differing in their rate of biodegradation that are compression moulded into a disc. The prolonged release of the botulinum toxin is therefore achieved by employing microspheres having a different rate of degradation in vivo. The polymers that may be used for the manufacture of the polymeric matrix can be selected from a wide group of materials (cf. US 6.312.708, column 20, lines 25 - 37), said materials having widely different properties such as glass transition temperature (T_{g}). For example, Alkermes, Inc. manufactures biodegradable polymers based on glycolic acid, lactide, caprolactone and mixtures thereof having glass transition temperatures within the range of -65° to 60° and melting points within the range of 60° to 230°C. The disadvantage of the system disclosed in US 6.312.708 is that it lacks controllability, i.e. that release of the drug cannot be initiated "at will" by some external activating source since it will inevitably release the drug in a continuous manner until all microspheres are degraded.

During the 2002 Annual Meeting of the American Institute of Chemical Engineers, an oral presentation was presented on November 6, 2002, at the Drug Delivery Session (15B12) of the Food, Pharmaceutical and Bioengineering Group (15). To that end an abstract was submitted that discloses in general terms the progress of a research project directed to the development of ultrasound-directed drug release from polymers such as polylactide and poly(lactide-co-glycolide). Ultrasound at a frequency of 1 MHz was applied to a polymeric slab in water and it appeared that the temperature of the polymer increased significantly, rapidly and linearly with ultrasonic intensity whereas the temperature of the surrounding - water was negligible. In the abstract it is suggested that "this fast temperature increase of the polymer can be utilised to change the glassy state of the polymer into a rubbery state by passing through the glass transition temperature, T_{g}, of the polymer". It is furthermore disclosed in the abstract that. "In the glassy state, below T_{g}, diffusion of incorporated substances is relatively low whereas in the rubbery state, above T_{g}, flexible polymer change allow for fast diffusion of incorporated drugs. Consequently, the glass transition temperature appears to be an important parameter to control ultrasound-induced drug release". However, the abstract does not provide an enabling disclosure of the intended drug delivery device. Nor does the abstract disclose which polymers were explicitly used.

US 4.657.543 and US 4.779.806 disclose a process for delivering a biologically active substance, e.g. drugs such as steroids, anti-cancer drugs and infection suppressing agents, on demand wherein a polymeric matrix in which the biologically active substance is encapsulated is exposed to ultrasound. The frequency and intensity of the ultrasound energy employed is such that the polymeric matrix is degraded by ultrasound induced cavitation so that the drug is released. Suitable frequencies are about 20 kHz to about 1000 kHz and a suitable power is in the range of about 1W to about 30 W. It is worth noting that in this patent specification reference is made to power (because of the unit W) and not to intensity (unit W/cm²) as employed in the present application. This method has the disadvantage that due to the irreversible degradation of the polymeric matrix release of the biologically active substance is difficult to control. Another disadvantage of this method is that only a small increase in the rate of release could be achieved (about a factor 10).

US 4.767.402 discloses a transdermal patch comprising a protective cover, a matrix containing a drug, a support and an adhesive. The transdermal patch is applied to the skin and release of the drug through the skin is induced by ultrasound energy having a frequency of 20 kHz to 10 MHz and an intensity of 0 to 3 W/cm². Instead of a patch, an aqueous gel can be used in which the drug is suspended to enhance the efficiency of the ultrasound energy since it does not transmit well through air. Usually, drugs are systemically administered by transdermal patches and higher concentrations can only be achieved when the location of administration is just subcutaneous. In addition, many drugs cannot be administered by this method since they will not pass the skin. Furthermore, the method suffers from the disadvantage that release on demand is difficult to control since patches and in particular aqueous gels do not adhere sufficiently to the skin for a prolonged period of time. Hence, for a patient it would be required to replace the patch on a regular basis, e.g. every two days.

US 5.947.921 and US 6.041.253 disclose a method for transdermal delivery of a drug by means of a combination of ultrasound and a magnetic field, electroporation or ionthophoresis.

EP 317.180 A1 discloses a temperature-controlled diffusional active agent dispenser that provides a reversible, substantial change in agent dispensing rate at a selected temperature comprising a solid polymer and an active agent. The polymer can be reversibly activated by conduction, induction or radiation. The permeability of the polymer is said to be influenced by T_{g} and T^{m}.

US 5.580.573 discloses a device for the controlled release of biologically active substances or other chemical substances. The device comprises an amorphous polymer and an active ingredient contained therein, wherein the amorphous polymer has a T_{g} of 20° - 50°C and the release of the active agent is activated upon heating the device to a temperature above the T_{g} of the polymer. According to an embodiment, the device constitutes of a combined monolithic reservoir system comprising a matrix of a polymer having a very low T_{g}, wherein the active ingredient is homogeneously dispersed and wherein said polymer matrix is surrounded by the release controlling polymer.

The present invention provides a drug delivery system that does not have the disadvantages of the prior art drug delivery systems. The advantages of the present invention are obtained by employing a method, wherein the transport characteristics, in particular of a drug delivery device, are modified in a reversible manner by supplying energy from an energy source in a manner that differs as a function of time. The transport characteristics are modified by employing certain polymeric materials having a T_{g} within a certain range, wherein heating the polymeric material above its T_{g} results in an enhanced diffusion of an active compound, in particular a drug, that is present in the polymeric material.

Within the framework of the present invention, the term "energy source" is to be understood to be a some of energy via (1) light waves, sound waves or microwaves, (2) electric current, (3) electrical, magnetic or radioactive radiation or (4) a combination thereof Also within the framework of the present invention, the term "active compound" is to be understood as a compound that provides, either directly or indirectly, a certain action. Preferably, the active compound provides a biological effect within a mammalian body. Hence, according to the invention, an active compound may be a medicament or pharmaceutical that has a direct biological effect on a mammalian body. But the invention is not regarded as limited to medicaments and pharmaceutical so that the term "active compound" also includeds substances like flavourings, odorants and colorants. Further, within the framework of the invention, the glass transition temperature T_{g} is to be understood as the glass transition temperature of the combination of solid polymeric material and active compound. That is, that the glass transition temperature as used herein does not refer to the glass transition temperature of the polymer *per se.*

### Summary of the invention

Accordingly, the present invention provides in particular a drug delivery device, in particular for implantation in a mammalian body, comprising a solid polymeric material having a barrier effect against diffusion of an active compound, and having a glass transition temperature within the range of about 0° to about 90°C, and an active compound, wherein the characteristics of diffusion or transport of the active compound can be modified in a reversible manner and the solid polymeric material is heated from a temperature below the glass transition temperature to a temperature above the glass transition temperature by supplying energy from an energy source, the energy source being selected from a source of energy via (1) light waves, sound waves or microwaves, (2) electric current, (3) electrical, magnetic or radioactive radiation or (4) a combination thereof, the drug delivery device comprising:
(a) (1) a core comprising the solid polymeric material and the active compound or (2) a material being permeable for the active compound and particles comprising the solid polymeric material and the active compound; and
(b) an enveloping material being thermally insulating and permeable for the active compound.

The present invention also relates to a method for releasing an active compound, from a drug delivery device, in particular for implantation in a mammalian body, the drug delivery device comprising a solid polymeric material and an active compound, wherein the active compound is released from the drug delivery device by reversibly modifying the transport characteristics of the solid polymeric material and heating the solid polymeric material from a temperature below the glass transition temperature to a temperature above the glass transition temperature by supplying energy from an energy source, the energy source being selected from a source of energy via (1) light waves, sound waves or microwaves, (2) electric current, (3) electrical, magnetic or radioactive radiation or (4) a combination thereof, and herein the solid polymeric material has a glass transition temperature within the range of about 0° to about 90°C.

### Detailed description of the invention

The present invention is based on the discovery that application of energy to a solid polymeric material results in absorption of the energy through viscous shearing and relaxation which results in heating of the solid polymeric material. It appears that, if the energy is ultrasound, that the temperature of the solid polymeric material increases linearly with the ultrasonic intensity. Moreover, the temperature increase is induced rapidly when the solid polymeric material is exposed to energy, e.g. ultrasound. This fast temperature increase is utilised to change the glassy state of the solid polymeric material into the rubbery state by heating the solid polymeric material from a temperature below the T_{g} of the solid polymeric material to a temperature above the T_{g} of the solid polymeric material. In addition, at a temperature around the T_{g} the absorption of energy, in particular ultrasonic energy, is substantial since mechanical damping has a maximum at this temperature. If the temperature of the solid polymeric material is below the T_{g}, release of the drug is extremely low whereas as the temperature of the solid polymeric material is above the T_{g}, flexible polymer chains allow for fast diffusion of the drug.

When the drug delivery device according to a preferred embodiment of the present invention is implanted in the body of a mammal, it is surrounded by an aqueous environment. In this aqueous environment, viscous shearing does not occur because energy, in particular ultrasound, is less absorbed by the water of the aqueous environment than by the solid polymeric material. Consequently, the drug delivery device according to the present invention does not have any harmful effect to a patient when it is exposed to energy such as ultrasound.

If the energy employed is ultrasound, the ultimate temperature increase that occurs within the solid polymeric material is dependent from the ultrasound frequency and the attenuation coefficient of the solid polymeric material. Hence, according to the invention, the solid polymeric material has preferably an attenuation at 1 MHz of about 10 to about 1000 dB/cm, more preferably about 50 to about 500 dB/cm.

An advantage of the drug delivery device and method according to the present invention is that the transport characteristics can be reversibly modified in such a way that the diffusion of the active compound occurs repeatedly and reproducibly. In this way, the release of an active compound can be controlled as a function of time. Another advantage of the present invention is that an active compound can be delivered on demand.

Depending on the therapeutic or diagnostic applications and the active compound concerned, the drug delivery device according to the invention can be made in various ways. According to a first, preferred embodiment, the active compound is mixed with the solid polymeric material having diffusion or transport characteristics that can be modified. According to a second, preferred embodiment, the active compound is in the centre of the drug delivery device and this centre is surrounded by the solid polymeric material for which the characteristics of diffusion or transport of the active compound can be modified in a reversible manner. In order to protect the tissues and/or the organs in which the drug delivery device is present from the energy supplied, it is preferable according to this preferred embodiment that the drug delivery device is surrounded by an enveloping material that is thermally insulating and is permeable for the active compound.

Accordingly, the preferred second embodiment of the present invention comprises a drug delivery device, in particular for implantation in a mammalian body, comprising:
a) a core comprising the solid polymeric material and the active compound; and
b) an enveloping material being thermally insulating and permeable for the active compound.

Even more preferably, the drug delivery device according to this preferred second embodiment comprises a core comprising a solid polymeric material in which the active compound is homogeneously dispersed. Alternatively, it is even more preferred that the drug device according to this second preferred embodiment has a core comprising an inert support comprising the active compound, wherein the inert support is coated with a layer of the solid polymeric material.

According to the invention, the enveloping material is preferably a hydrogel. Hydrogels are three dimensional networks of hydrophilic polymers in which a large amount of water is present. In general the amount of water present in a hydrogel is at least 20 weight percent of the total weight of the dry polymer. The most characteristic property of these hydrogels is that it swells in the presence of water and shrinks in the absence of water. The extent of swelling (equilibrium water content) is determined by the nature (mainly the hydrophilicity) of the polymer chains and the crosslinking density. Polymers than can be used for manufacturing hydrogels are well known in the art and are for example disclosed in US 2.340.110, US 2.340.111, US 2.533.635, US 2.798.053, US 3.940.351, US 4.062.817, US 5.034.486, US 5.034.487, US 5.034.488 and US 5.468.797 .

According to another more preferred embodiment of the present invention, the drug delivery device comprises a material being permeable for the active compound and particles comprising the solid polymeric material and the active compound, wherein the material being permeable for the active compound is heated by supplying energy from an energy source. Preferably, the material being permeable for the active compound is coated with an enveloping material being thermally insulating and permeable for the active compound. The advantage of this preferred embodiment is that the environment in which the drug delivery device is present and in which the method is thus employed, in particular the physiological environment of the mammalian body, can be protected from changes in the drug delivery device that are a consequence of the supply of energy. One example of this is protection from a rise in the temperature of the drug delivery device.

In addition, the use of an enveloping material being thermally insulating and permeable for the active compound has as further advantages that it counteracts fouling of the solid polymeric material. It can further provide more strength to the drug delivery device and provide flexibility thereto and inhibits negative influences from sarcophagus and aggressive bacteria. It further provides the option that solid polymeric materials can be used that are less biocompatible (rejections) provided that the enveloping material is sufficiently biocompatible.

Research has revealed that particular good results are obtained when a poly(lactic acid-co-glycolic acid), polymethyl methacrylate, poly(ethylene-co-vinyl acetate) or a nylon is used as the solid polymeric material. However, it is even more preferred that the solid polymeric material comprises a polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxane-2-one) and mixtures thereof. Preferably, the copolymer is a random copolymer or a block copolymer and the block copolymer is preferably a diblock copolymer or a triblock copolymer. Such polymers and copolymers are well known in the art and are for example disclosed in US 2.668.162, US 2.703.316, US 3.636.956, US 3.839,297, US 4.137.921, US 4.157.437, US 4.243.775, US 4.443.430, US 5.076.983, US 5.310.865 and US 6.025.458. This group of even more preferred solid polymeric materials also includes (meth)acrylic (co)polymers, polyester urethanes, polyester amides, polyether esters such as polyethylene glycol terephtalate - polybutylene terephtalate (PEGT/PBT), polyethylene glycol and the natural polymers such as polyhydralonic acid, iso-sorbide, dextran, collagens and mixtures thereof. Another even more preferred solid polymeric material is also polybutyl methacrylate.

In principle, the present invention can be employed for modifying the transport characteristics of any solid polymeric material that has a barrier effect against diffusion for an active compound. Particularly advantageous applications of the present invention are in the field of the release of active compounds. Preferably, active compounds such as medicaments, diagnostic agents and contrast media for imaging are incorporated in the solid polymeric material. If the active compound is a medicament, it is preferred that the medicament is selected from the group consisting of chemotherapeutic agents, analgesics and anaesthetics, hormonal substances, infection suppressing agents, and antiarrhythamic agents.

The present invention also relates to a method for releasing an active compound from a drug delivery device according to the present invention, wherein it is preferred that the drug delivery device is for implantation in a mammalian body. However, other uses are also suitable and are disclosed in this patent application as well.

Methods for releasing an active substance are generally known in the field of medicine. Drug delivery devices comprising an active compound that has to be released in a controlled manner as a function of time are usually based on the fact that the active compound is incorporated in a material that slowly releases the active compound by diffusion. However, a common feature of all of these known methods is that, irrespectively of the mechanisms on which the release of the active compound are based, the release can no longer be stopped after it has started. Therefore, the known methods are irreversible.

An advantage of the method according to the invention is that an active compound can be repeatedly released from the drug delivery device in a controlled manner as a function of time, depending on the therapeutic or diagnostic needs. It is preferred that a medicament selected from the group of chemotherapeutic agents, analgesics and anaesthetics, hormonal substances, infection suppressing agents and antiarrhythamic agents is used as active compound. It is likewise preferred that a diagnostic agent or a contrast medium for imaging is used as active compound.

Preferably, the energy is supplied pulse-wise so that the transport characteristics are also modified pulse-wise, with the result that the active compound is not released continuously but is also released pulse-wise by diffusion as a result of the interin re-emergence of the barrier effect For example, if the active compound is an analgesic, a patient in need thereof can induce the release of the analgesic on demand by switching on the energy source.

According to a preferred embodiment of the present invention, the energy is supplied from an energy source that is external with respect to the drug delivery device. According to this invention, the transport characteristics of the polymer can be modified remotely.

According to the invention, the energy may origin from many sources as explained above, but it is preferred that the energy source is a source for ultrasound, infra red radiation or magnetic energy, or a combination thereof.

If the energy to be employed is magnetism, the solid polymeric material must contain a magnetic material, for example solid magnetic particles as are disclosed in Chan et al., "Scientific and Clinical Applications of Magnetic Carriers", Häfeli et al. (Eds.), pages 607 - 617 Plenum Press, 1997. Alternatively, nanotubes may be included to render the solid polymeric material conductive so that when a magnetic field is applied an induced current is generated. As a result of the resistance that is encountered by this induced current, the solid polymeric material is heated (cf. Iijima, S., Helical microtubes of graphitic carbon, Nature, Vol. 354, 56 - 58; Iijima, S., Ichihasi, T., Single-shell carbon nanotubes of I-nm diameter, Nature, Vol 363, 1993, 603 - 605; Lambin, P., Electronic structure of carbon nanotubes, C.R. Physique, Vol. 4, 2003, 1009 -1019; Dresselhaus, M., Dresslhaus, G., Eklund, P., Saito, R., Carbon nanotubes, Physics World, Vol. 11, Issue 1, 1998, 33 - 38; Kiang, C., Goddard, W.A., Beyers, R., Bethune, D.S., Carbon nanotubes with single-layer walls, Carbon, Vol 33, Issue 7, 903 - 914 ).

The ultimate temperature increase that occurs within the magnetic material is measured in the Specific Power Absorption Rate (SAR) - cf. Chan *et al. -* wherein the optimum ranges are within the range of 500 - 600 W/g. According to the invention, it is preferred that about 3 to about 30 mg, preferably about 5 to about 15 mg, solid magnetic particles are incorporated in the solid polymeric material per g solid polymeric material.

If the energy to be employed is infra red light, the ultimate temperature increase that occurs within the solid polymeric material is dependent on the intensity and wavelength of the infra red light as will be understood by the person skilled in the art. Obviously, a specific inter-atomic bond of the polymer can for example be activated by using a specific wave length.

According to a preferred embodiment of the method according to the invention, a source of ultrasonic sound is used as energy source. This particular method is very suitable for remote use, where contact with an object (and the environment thereof) is avoided. According to particularly advantageous application variants, the ultrasonic sound has a frequency of 2 x 10⁴ Hz to 1 x 10⁷ Hz (20 kHz to 10 MHz), more preferably 5 x 10⁵ Hz to 5 x 10⁶ Hz (500 kHz to 5 MHz). In order to protect the surrounding environment from the supply of energy by ultrasonic sound waves, it is preferable that the sound waves are focussed in a beam.

It is also preferred that the ultrasound has an intensity of 0.1 to 20.0 W/cm², preferably 0.2 to 5.0 W/cm².

Experiments have further shown that it is preferred that the external energy source is used in such a way that the solid polymeric material is subjected to one or more, more preferably two or more, reversible glass transitions between a glassy state and a rubbery state. It has been found that such transitions, which as such require relatively little energy, can lead to very appreciable modifications in the transport characteristics, which, in turn, can lead to a very appreciable increase in the diffusion of an active compound present in the material. According to a particularly preferred application, the glass transition or glass transitions occur at a temperature of about 30° to about 90°C, more preferably about 35° to about 80°C, even more preferably about 35 to about 65 °C, yet even more preferably about 35 to about 60°, yet even more preferably about 37 to about 55°C and most preferably about 40 to about 55°C. Experiments show that these temperature ranges are adequate to obtain the particular advantages according to the present invention.

According to the preferred embodiment of the method for releasing an active compound from a drug delivery device according to the invention, the energy is supplied pulse-wise. Moreover, it is preferred that the energy is supplied from an energy source that is external with respect to the material. The advantages of these preferred embodiments are that the release of the active compound(s) can be controlled pulse-wise and that the energy can be supplied remotely from the patient by an intervention of the physician, non-academic hospital personnel such as nurses, or the patient himself, respectively. Depending on the nature of the complaint to be treated and the characteristics of the drug delivery device employed and the active compound present therein, the physician or the patient can regulate the duration, the frequency and the intensity of the release of the active compound. Preferably, the energy source is used in such a way that the material is subjected to one or more, preferably two or more, reversible glass transitions between a glassy state and a rubbery state.

The drug delivery device according to the present invention may for example be used to control post-operative heart rhythm complaints wherein the drug delivery device including the appropriate medicament is implanted in the pericardium.

Another embodiment of the present invention is a carrier, preferably a catheter, provided with a layer of the solid polymeric material, wherein the solid polymeric material comprises an active compound, preferably an infection suppressing medicament, wherein the carrier is also provided with a heating means. Such catheters are for example disclosed in US 6.740.108.

According to yet another embodiment of the invention, particles comprising the solid polymeric material and the active compound can occur in a form that can be administered by injection, e.g. intravenously or intramuscularly, as is for example disclosed in US 2003/0212148.

The present invention further relates to the use of a solid polymeric material as defined herein to release an active compound, wherein the diffusion or transport characteristics of the solid polymeric material for the active compound can be modified by supplying energy from an energy source, in particular an external energy source, the solid polymeric material having a glass transition temperature within the range of about 0° to about 90°C. In such a use, the active compound is not limited to medicaments, diagnostic agents and contrast media for imaging, but may also be selected from compounds such as flavourings, odorants or colourants. For example, an interesting application would be the release of flavourings in cooled fresh meals so that when the cooled fresh meals are taken out from the fridge the flavourings are released as soon as the fresh meal reaches a temperature of about room temperature.

### Examples

### Comparative example 1

An object consisting of a pressed circular disc with a diameter of 2 cm and thickness of 2 mm was placed in a holder in a vessel containing 0.5 liter physiological buffer. The disc consisted of poly(butyl methacrylate-co-methyl methacrylate), or p(BMA-MMA), with 75 mol% of pBMA, in which copolymer the pharmaceutical active compound ibuprofen had been incorporated in a mass ratio of 5 % (m/m). The vessel was first placed in a shaking bad of 20 °C, where the release rate of ibuprofen into the physiological buffer was determined as a function of time. Then the vessel was placed in a shaking bath of 50 °C and again the speed of the release of ibuprofen into the physiological buffer was determined as a function of time. This series was repeated 5 times. At 20 °C the release rate was 0,36 nmol/h, at 50 °C the release rate was 10 mnol/h.

This experiment is repeated with poly(lactic acid-co-glycolic acid), or PLAGA, with a ratio of lactic acid to glycolic acid of 50/50, both with ibuprofen incorporated The glass transition temperature of the matrix of poly(lactic acid-co-glycolic acid) used was 43 °C.

This resulted in a similar difference in release rate between 20 °C and 50 °C as in the previous example.

The experiment is also repeated with naproxen, lidocaïne, gentamicine and fentanyl as the pharmaceutical active compound, also resulting in a similar difference in release rate between 20 °C and 50 °C.

### Comparative example 2

### Example 2a

An object consisting of a pressed circular disc with a diameter of 2 cm and thickness of 2 mm was provided with a thermocouple in the middle and was then placed in a holder in a vessel containing 0.5 litre physiological buffer. The disc consisted of poly(lactic acid-*co*-glycolic acid), or PLAGA, with a ratio of lactic acid to glycolic acid of 50/50, in which copolymer the pharmaceutical active compound ibuprofen had been incorporated in a mass ratio of 10 % (m/m). The PLAGA was purchased from Purac Biochem, The Netherlands. The glass transition temperature of the matrix of poly(lactic acid-co-glycolie acid) used was 12.7 °C (as compared to Example 1 where a T_{g} of 43°C is disclosed. This decrease of the T_{g} is the result of a higher loading of ibuprofen).

The disc was irradiated perpendicularly to the surface with a beam of ultrasonic sound with a power of 4 W and a frequency of 10⁶ Hz (1 MHz), the probe for generating the ultrasound being 28 mm away from the disc. The speed of the release of ibuprofen into the physiological buffer was determined as a function of time. Both the temperature of the buffer and the temperature in the disc were measured continuously. The irradiation with ultrasonic sound was applied in an alternating manner, periods of irradiation of 120 minutes being alternated with periods of 60 minutes without irradiation.

As a consequence of the irradiation, the temperature of the buffer in each case rose from 8 °C to 11 °C; the temperature of the disc in each case rose to 18 °C. After the irradiation, the temperature of the buffer and of the disc fell back to 8 °C. As a consequence of the irradiation, the speed of release of ibuprofen from the disc in each case increased from less than 1 x 10⁻⁷ gram/minute to values fluctuating between 1.1 x 10⁻⁵ gram/minute to 1.7 x 10⁻⁵ gmm/minute. After stopping the irradiation, the release of ibuprofen in each case fell virtually immediately to the base value.

Similar results were obtained using a matrix of isotactic polymethyl methacrylate instead of poly(lactic acid-co-glycolic acid).

### Example 2b

In a vessel poly(hydroxyethyl methacrylate), or HEMA, and deionised water were premixed in a mass ratio of 95/5 and a total volume of 100 ml. While stirring, EDMA (Ethylene DiMethAcrylate) is added as a cross-linking agent. A 10% solution of ammonium persulphate (APS) and pure tetramethylethylene diamine (TEMED) are used as initiator couple. After adding the initiator couple, the magnetic stirrer is removed and the vessel is covered with parafilm. Polymerization was carried out at room temperature for 24 hours. After polymerization the spongy material is placed in deionised water to remove unreacted monomer and initiator.

The sponge material was cut open and a p(BMA-MMA) tablet, similar as used in comparative example 1, was placed inside the material.

The combination of HEMA and the disc were placed in a vessel containing 0,5 liter physiological buffer and irradiated perpendicularly to the surface with a beam of ultrasonic sound, with a power of 1,7 W and a frequency of 10⁶ Hz (1 MHz), the probe for generating the ultrasound being 20 mm away from the disc. The irradiation with ultrasonic sound was applied in an alternating manner, periods of irradiation of 30 minutes being alternated with periods of 60 minutes without irradiation.

During the experiments the temperatures were measured of the core of the disc, of the space in between the surface of the disc and the layer of HEMA (at both sides of the disc), of the core of the layer of HEMA (at both sides of the disc) and of the physiological buffer. On average, the temperature of the disc increased with 31 °C, whereas the average temperature of the HEMA increased with only 7°C. The temperature of the physiological buffer increased with 2 °C.

### Example 2c

A pressed circular disc of p(BMA-MMA) with ibuprofen incorporated in the matrix with a mass ratio of 3% (m/m), as described in comparative example 1, was implanted subcutaneously in a Lewis rat. On both sides of the disc a thermocouple was implanted to continuously measure the temperature at the surface of the disc. One hour after implantation the disc was irradiated perpendicularly to the surface with a beam of ultrasonic sound with a power of 0,7 W, a frequency of 10⁶ Hz (1 MHz), and a duty cycle of 70%, the probe for generating the ultrasound touching the skin of the rat. A standard physiotherapeutic hydrogel was used in between the skin and the probe. The irradiation with ultrasonic sound was applied in an alternating manner, periods of irradiation of 30 minutes being alternated with periods of 30 minutes without irradiation.

Blood samples were taken each 10 minutes during irradiation and each 20 minutes in the periods without irradiation to determine concentration of ibuprofen in the blood stream as a function of time. After 30 minutes irradiation the concentration of ibuprofen was 0,9 µg/ml, after irradiation the plasma concentration went below 0,01 µg/ml.

The temperature nearby the surface of the disc was measured continuously at both sides; the value ranged between 35 and 41 °C. Also the rectal temperature of the rat was measured and no significant changes in temperature were found

### Comparative example 3

Discs of poly(butyl-co-methylmethacrylate) were loaded with 5 mg of magnetic material per gram of polymer. The magnetic material used consisted of dextran-coated iron oxide particles with an effective hydrodynamic diameter of 5 - 50 nm and was synthesized following the procedure described in Chan, D.C.F., Kirpotin, D.B., Bunn, P.A., Physical chemistry and In Vivo tissue heating properties of colloidal magnetic iron oxides with increased power absorption rates, Scientific and Clinical Applications of Magnetic Carriers, Häfeli et al., Plenum Press, New York, 1997.

Discs of this loaded polymer of 2 cm diameter and 5 mm thickness were placed in a vessel containing 0.5 liter physiological butter of 37 °C and were subjected to a 1 MHz AC magnetic field of 8 kA/m (~ 100 Oersted). The core temperature of the disc was measured to increase by 8 to 10 °C within 5-10 minutes. When the magnetic field was turned off, the temperature of the disc returned to 37 °C within 5 minutes.

Similar results were obtained when poly(methyl methacrylate) or poly(lactic acid-co-glycotic acid) was used.

## Claims

1. A drug delivery device comprising a solid polymeric material having a barrier effect against diffusion of an active compound and having a glass transition temperature within the range of 0° to 90°C and an active compound, wherein the characteristics of diffusion or transport of the active compound can be modified in a reversible manner and the solid polymeric material is heated from a temperature below the glass transition temperature to a temperature above the glass transition temperature by supplying energy from an energy source, the energy source being selected from a source of energy via (1) light waves, sound waves or microwaves, (2) electric current, (3) electrical, magnetic or radioactive radiation or (4) a combination thereof, the drug delivery device comprising:
(a) (1) a core comprising the solid polymeric material and the active compound or (2) a material being permeable for the active compound and particles comprising the solid polymeric material and the active compound; and
(b) an enveloping material being thermally insulating and permeable for the active compound.

2. Drug delivery device according to Claim 1, wherein the core comprises the solid polymeric material in which the active compound is homogeneously dispersed.

3. Drug delivery device according to Claim 1, wherein the core comprises an inert support comprising the active compound, wherein the inert support is coated with a layer of the solid polymeric material.

4. Drug delivery device according to any one of Claims 1 - 3, wherein the enveloping material is a hydrogel.

5. Drug delivery device according to any one of Claims 1 - 4, wherein the solid polymeric material comprises a polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxano-2-one) and mixtures thereof, and/or wherein the solid polymeric material comprises a polymer selected from the group consisting of (meth)acrylic (co)polymers, polyester urethanes, polyester amides, polyether esters such as polyethylene glycol terephtalate - polybutylene terephalate (PEGT/PBT), polyethylene glycol and the natural polymers such as polyhydralic acid, iso-sorbide, dextran, collagens and mixtures thereof.

6. Drug delivery device according to any one of Claims 1 - 5, wherein the active compound is selected from the group consisting of medicaments, diagnostic agents and contrast media for imaging.

7. Drug delivery device according to Claim 6, wherein the medicament is selected from the group consisting of chemothctapeutic agents, analgesics, anaesthetics, hormonal substances, infection suppressing agents and antiarrhythamic agents.

8. Drug delivery device according to any one of Claims 1 - 7, wherein the energy source is a source for ultrasound, infrared radiation, magnetic energy, or a combination thereof.

9. A method for releasing an active compound from a drug delivery device according to any one of Claims 1 - 8, the drug delivery device comprising a solid polymeric material and an active compound, wherein the active compound is released from the drug delivery device by reversibly modifying the transport characteristics of the solid polymeric material and heating the solid polymeric material from a temperature below the glass transition temperature to a temperature above the glass transition temperature by supplying energy from an energy source, the energy source being selected from a source of energy via (1) light waves, sound waves or microwaves, (2) electric current, (3) electrical, magnetic or radioactive radiation or (4) a combination thereof, and wherein the solid polymeric material has a glass transition temperature within the range of 0° to 90°C.

10. Method according to Claim 9, wherein the energy is supplied pulse-wise.

11. Method according to Claim 9 or Claim 10, wherein the energy is supplied from an energy source that is external with respect to the solid polymeric material.

12. Method according to any one of Claims 9 to 11, wherein the energy source is a source for ultrasound, infrared radiation, magnetic energy or a combination thereof.

13. Method according to any one of Claims 9 - 12, wherein the active compound is selected from the group consisting of medicaments, diagnostic agents and contrast media for imaging.

14. Method according to Claim 13, wherein the medicament is selected from the group consisting of chemotherapeutic agents, analgesics, anaesthetics, hormonal substances, infection suppressing agents and antiarrhythamic agents.

15. Method according to any one of Claims 9 - 14, wherein the solid polymeric material comprises a biodegradable polymer or a copolymer comprising a monomer selected from the group consisting of a hydroxyl alkanoate wherein the alkyl group comprises 1 to 12 carbon atoms, lactide, glycolide, ε-caprolactone, 1,4-dioxane-2-one, 1,5-dioxepan-2-one, trimethylene carbonate (1,3-dioxane-2-one) and mixtures thereof, and/or wherein the solid polymeric material comprises a polymer selected from the group consisting of (nteth)acrylic (co)polymers, polyester urethanes, polyester amides, polyether esters such as polyethylene glycol terephtalate - polybutylene terephalate (PEGT/PBT), polyethylene glycol and the natural polymers such as polyhydralonic acid, iso-sorbide, dextran, collagens and mixtures thereof.

16. Use of the drug delivery device according to any one of Claims 1 - 8 for implantation in a mammalian body.

17. Use of the drug delivery device according to any one of Claims 1 - 8 for incorporation into a prosthesis or an artificial organ.

18. Carrier provided with a layer of a solid polymeric material as defined in any one of Claims 1 - 8, wherein the solid polymeric material comprises an active compound, wherein the carrier is also provided with a heating means.

19. Carrier according to Claim 18, wherein the carrier is a catheter.

20. Carrier according to Claim 18 or Claim 19, wherein the active compound is an infection suppressing medicament.

## Patentansprüche

1. Arzneimittelzufuhrvorrichtung, umfassend einen festen Polymerstoff mit einer Schrankenwirkung gegenüber Diffusion eines Wirkstoffes und mit einer Glasübergangstemperatur innerhalb des Bereiches von 0° bis 90° C und einen Wirkstoff, wobei die Diffusions- oder Transporteigenschaften des Wirkstoffs auf reversible Weise modifiziert werden können und wobei der feste Polymerstoff von einer Temperatur unterhalb der Glasübergangstemperatur auf eine Temperatur oberhalb der Glasübergangstemperatur erhitzt wird, indem Energie von einer Energiequelle zugeführt wird, wobei die Energiequelle ausgewählt ist aus einer Energiequelle über (1) Lichtwellen, Tonwellen oder Mikrowellen, (2) elektrischen Strom, (3) elektrische, magnetische oder radioaktive Strahlung oder (4) eine Kombination von diesen, wobei die Arzneimittelzufuhrvorrichtung umfasst:
(a) (1) einen Kern, der den festen Polymerstoff und den Wirkstoff umfasst oder (2) einen Stoff, der permeabel für den Wirkstoff ist, und Partikel die den festen Polymerstoff und den Wirkstoff umfassen; und
(b) einen Hüllstoff, der thermisch isolierend und für den Wirkstoff permeabel ist.

2. Arzneimittelzufuhrvorrichtung nach Anspruch 1, wobei der Kern den festen Polymerstoff umfasst, in dem der Wirkstoff homogen verteilt ist.

3. Arzneimittelzufuhrvorrichtung nach Anspruch 1, wobei der Kern einen inerten Träger umfasst, der den Wirkstoff umfasst, wobei der inerte Träger mit einer Schicht des festen Polymerstoffes beschichtet ist.

4. Arzneimittelzufuhrvorrichtung nach einem der Ansprüche 1 bis 3, wobei der Hüllstoff ein Hydrogel ist.

5. Arzneimittelzufuhrvorrichtung nach einem der Ansprüche 1 bis 4, wobei der feste Polymerstoff ein Polymer oder ein Copolymer umfasst, das ein Monomer umfasst, ausgewählt aus der Gruppe bestehend aus einem Hydroxylalkanoat wobei die Alkylgruppe 1 bis 12 Kohlenstoffatome umfasst, Lactid, Glycolid, ε-Caprolacton, 1,4-Dioxan-2-on, 1,5-Dioxepan-2-on, Trimethylencarbonat (1,3-Dioxan-2-on) und Gemische davon, und/oder wobei der feste Polymerstoff ein Polymer umfasst, ausgewählt aus der Gruppe bestehend aus (Meth)Acryl(Co)Polymeren, Polyesterurethanen, Polyesteramiden, Polyetherestern wie Polyethylen-glycol-terephtalat - Polybutylen-terephtalat (PEGT/PBT), Polyethylenglycol und die natürlichen Polymere wie Polyhydralonsäure, Iso-Sorbid, Dextran, Kollagene und Gemische davon.

6. Arzneimittelzufuhrvorrichtung nach einem der Ansprüche 1 bis 5, wobei der Wirkstoff ausgewählt ist aus der Gruppe bestehend aus Arzneimitteln, Diagnosemitteln und Kontrastmedien zur Bildgebung.

7. Arzneimittelzufuhrvorrichtung nach Anspruch 6, wobei das Arzneimittel ausgewählt ist aus der Gruppe bestehend aus chemotherapeutischen Mitteln, Analgesica, Anaesthetica, hormonellen Substanzen, infektionsunterdrückenden Mitteln und antiarrhythmischen Mitteln.

8. Arzneimittelzufuhrvorrichtung nach einem der Ansprüche 1 bis 7, wobei die Energiequelle eine Quelle für Ultraschall, Infrarotstrahlung, magnetische Energie oder eine Kombination von diesen ist.

9. Verfahren zur Freisetzung eines Wirkstoffes von einer Arzneimittelzufuhrvorrichtung nach einem der Ansprüche 1 bis 8, wobei die Arzneimittelzufuhrvorrichtung einen festen Polymerstoff und einen Wirkstoff umfasst, wobei der Wirkstoff von der Arzneimittelzufuhrvorrichtung freigesetzt wird, indem die Transporteigenschaften des festen Polymerstoffes reversibel modifiziert werden und der feste Polymerstoff von einer Temperatur unterhalb der Glasübergangstemperatur auf eine Temperatur oberhalb der Glasübergangstemperatur erwärmt wird, indem Energie von einer Energiequelle zugeführt wird, wobei die Energiequelle ausgewählt ist von einer Energiequelle über (1) Lichtwellen, Tonwellen oder Mikrowellen, (2) elektrischen Strom, (3) elektrische, magnetische oder radioaktive Strahlung oder (4) eine Kombination von diesen, und wobei der feste Polymerstoff eine Glasübergangstemperatur innerhalb des Bereiches von 0° bis 90° C hat.

10. Verfahren nach Anspruch 9, wobei die Energie pulsweise zur Verfügung gestellt wird.

11. Verfahren nach Anspruch 9 oder Anspruch 10, wobei die Energie von einer Energiequelle zugeführt wird, die bezüglich des festen Polymerstoffes extern ist.

12. Verfahren nach einem der Ansprüche 9 bis 11, wobei die Energiequelle eine Quelle für Ultraschall, Infrarotstrahlung, magnetische Energie oder eine Kombination von diesen ist.

13. Verfahren nach einem der Ansprüche 9 bis 12, wobei der Wirkstoff ausgewählt ist aus der Gruppe bestehend aus Arzneimitteln, Diagnosemitteln und Kontrastmitteln zur Bildgebung.

14. Verfahren nach Anspruch 13, wobei das Arzneimittel ausgewählt ist aus der Gruppe bestehend aus chemotherapeutischen Mitteln, Analgesica, Anaesthetica, hormonellen Substanzen, infektionsunterdrückenden Mitteln und antiarrhythmischen Mitteln.

15. Verfahren nach einem der Ansprüche 9 bis 14, wobei der feste Polymerstoff ein biologisch abbaubares Polymer oder ein Copolymer umfasst, umfassend ein Monomer ausgewählt aus der Gruppe bestehend aus einem Hydroxylalkanoat, wobei die Alkylgruppe 1 bis 12 Kohlenstoffatome umfasst, Lactid, Glycolid, ε-Caprolacton, 1,4-Dioxan-2-on, 1,5-Dioxepan-2-on, Trimethylencarbonat (1,3-Dioxan-2-on) und Gemische davon, und/oder wobei der feste Polymerstoff ein Polymer umfasst, ausgewählt aus der Gruppe bestehend aus (Meth)Acryl(Co)Polymeren, Polyesterurethanen, Polyesteramiden, Polyetherestern wie Polyethylen-glycol-terephtalat - Polybutylen-terephtalat (PEGT/PBT), Polyethylenglycol und die natürlichen Polymere wie Polyhydralonsäure, Iso-Sorbid, Dextran, Kollagene und Gemische davon.

16. Verwendung einer Arzneimittelzufuhrvorrichtung nach einem der Ansprüche 1 bis 8 zur Implantation in einem Säugetierkörper.

17. Verwendung einer Arzneimittelzufuhrvorrichtung nach einem der Ansprüche 1 bis 8 zum Einbau in eine Prothese oder ein künstliches Organ.

18. Träger ausgestattet mit einer Schicht eines festen Polymerstoffes, wie in den Ansprüchen 1 bis 8 definiert, wobei der feste Polymerstoff einen Wirkstoff umfasst und wobei der Träger mit einem Heizmittel ausgestattet ist.

19. Träger nach Anspruch 18, wobei der Träger ein Katheter ist.

20. Träger nach Anspruch 18 oder Anspruch 19, wobei der Wirkstoff ein infektionsunterdrückendes Arzneimittel ist.

## Revendications

1. Dispositif de délivrance de substance médicamenteuse, comprenant une matière polymère solide ayant un effet barrière contre la diffusion d'un composé actif et ayant une température de transition vitreuse dans la gamme de 0 ° à 90 °C et un composé actif, dans lequel les caractéristiques de diffusion ou de transport du composé actif peuvent être modifiées d'une manière réversible et la matière polymère solide est chauffée depuis une température en dessous de la température de transition vitreuse jusqu'à une température au-dessus de la température de transition vitreuse en apportant de l'énergie depuis une source d'énergie, la source d'énergie étant choisie parmi une source d'énergie passant par (1) les ondes lumineuses, les ondes sonores ou les micro-ondes, (2) un courant électrique, (3) un rayonnement électrique, magnétique ou radioactif ou (4) une combinaison de ceux-ci, le dispositif de délivrance de substance médicamenteuse comprenant :
(a) (1) un coeur comprenant la matière polymère solide et le composé actif ou (2) une matière qui est perméable au composé actif et des particules comprenant la matière polymère solide et le composé actif ; et
(b) une matière d'enveloppe qui est isolante thermiquement et perméable au composé actif.

2. Dispositif de délivrance de substance médicamenteuse selon la revendication 1, dans lequel le coeur comprend la matière polymère solide dans laquelle le composé actif est dispersé de façon homogène.

3. Dispositif de délivrance de substance médicamenteuse selon la revendication 1, dans lequel le coeur comprend un support inerte comprenant le composé actif, dans lequel le support inerte est revêtu avec une couche de la matière polymère solide.

4. Dispositif de délivrance de substance médicamenteuse selon l'une quelconque des revendications 1 à 3, dans lequel la matière d'enveloppe est un hydrogel.

5. Dispositif de délivrance de substance médicamenteuse selon l'une quelconque des revendications 1 à 4, dans lequel la matière polymère solide comprend un polymère ou un copolymère comprenant un monomère choisi dans le groupe constitué par un hydroxyl alcanoate dans lequel le groupe alkyle comprend 1 à 12 atomes de carbone, un lactide, un glycolide, la ε-caprolactone, la 1,4-dioxane-2-one, la 1,5-dioxépan-2-one, le carbonate de triméthylène (1,3-dioxane-2-one) et des mélanges de ceux-ci, et/ou dans lequel la matière polymère solide comprend un polymère choisi dans le groupe constitué par les (co)polymères (méth)acryliques, les polyester uréthanes, les polyester amides, les polyéther esters tels qu'un polyéthylèneglycol téréphtalate-polybutylène téréphtalate (PEGT/PBT), un polyéthylèneglycol et les polymères naturels tels qu'un acide polyhydralonique, un isosorbide, le dextrane, les collagènes et des mélanges de ceux-ci.

6. Dispositif de délivrance de substance médicamenteuse selon l'une quelconque des revendications 1 à 5, dans lequel le composé actif est choisi dans le groupe constitué par les médicaments, les agents diagnostiques et les milieux de contraste pour imagerie.

7. Dispositif de délivrance de substance médicamenteuse selon la revendication 6, dans lequel le médicament est choisi dans le groupe constitué par les agents chimiothérapeutiques, les analgésiques, les anesthésiques, les substances hormonales, les agents suppresseurs d'infection et les agents anti-arythmiques.

8. Dispositif de délivrance de substance médicamenteuse selon l'une quelconque des revendications 1 à 7, dans lequel la source d'énergie est une source pour un ultrason, un rayonnement infrarouge, une énergie magnétique, ou une combinaison de ceux-ci.

9. Procédé pour libérer un composé actif à partir d'un dispositif de délivrance de substance médicamenteuse selon l'une quelconque des revendications 1 à 8, le dispositif de délivrance de substance médicamenteuse comprenant une matière polymère solide et un composé actif, dans lequel le composé actif est libéré à partir du dispositif de délivrance de substance médicamenteuse en modifiant de façon réversible les caractéristiques de transport de la matière polymère solide et en chauffant la matière polymère solide depuis une température en dessous de la température de transition vitreuse jusqu'à une température au-dessus de la température de transition vitreuse en fournissant de l'énergie à partir d'une source d'énergie, la source d'énergie étant choisie parmi une source d'énergie passant par (1) les ondes lumineuses, les ondes sonores ou les micro-ondes, (2) un courant électrique, (3) un rayonnement électrique, magnétique ou radioactif ou (4) une combinaison de ceux-ci, et dans lequel la matière polymère solide a une température de transition vitreuse dans la gamme de 0 ° à 90 °C.

10. Procédé selon la revendication 9, dans lequel l'énergie est apportée par impulsions.

11. Procédé selon la revendication 9 ou la revendication 10, dans lequel l'énergie est fournie à partir d'une source d'énergie qui est externe par rapport à la matière polymère solide.

12. Procédé selon l'une quelconque des revendications 9 à 11, dans lequel la source d'énergie est une source pour un ultrason, un rayonnement infrarouge, une énergie magnétique ou une combinaison de ceux-ci.

13. Procédé selon l'une quelconque des revendications 9 à 12, dans lequel le composé actif est choisi dans le groupe constitué par les médicaments, les agents diagnostiques et les milieux de contraste pour imagerie.

14. Procédé selon la revendication 13, dans lequel le médicament est choisi dans le groupe constitué par les agents chimiothérapeutiques, les analgésiques, les anesthésiques, les substances hormonales, les agents suppresseurs d'infection et les agents anti-arythmiques.

15. Procédé selon l'une quelconque des revendications 9 à 14, dans lequel la matière polymère solide comprend un polymère biodégradable ou un copolymère comprenant un monomère choisi dans le groupe constitué par un hydroxyl alcanoate dans lequel le groupe alkyle comprend 1 à 12 atomes de carbone, un lactide, un glycolide, la ε-caprolactone, la 1,4-dioxane-2-one, la 1,5-dioxépan-2-one, le carbonate de triméthylène (1,3-dioxane-2-one) et des mélanges de ceux-ci, et/ou dans lequel la matière polymère solide comprend un polymère choisi dans le groupe constitué par les (co)polymères (méth)acryliques, les polyester uréthanes, les polyester amides, les polyéther esters tels qu'un polyéthylèneglycol téréphtalate-polybutylène téréphtalate (PEGT/PBT), un polyéthylèneglycol et les polymères naturels tels qu'un acide polyhydralonique, un isosorbide, le dextrane, les collagènes et des mélanges de ceux-ci.

16. Utilisation du dispositif de délivrance de substance médicamenteuse selon l'une quelconque des revendications 1 à 8, pour l'implantation dans un corps de mammifère.

17. Utilisation du dispositif de délivrance de substance médicamenteuse selon l'une quelconque des revendications 1 à 8, pour l'incorporation dans une prothèse ou un organe artificiel.

18. Support doté d'une couche d'une matière polymère solide telle que définie dans l'une quelconque des revendications 1 à 8, dans lequel la matière polymère solide comprend un composé actif, dans lequel le support est aussi doté d'un moyen chauffant.

19. Support selon la revendication 18, dans lequel le support est un cathéter.

20. Support selon la revendication 18 ou la revendication 19, dans lequel le composé actif est un médicament suppresseur d'infection.
